(19) Europäisches Patentamt
European Patent Office
Office européen des brevets

(11) Veröffentlichungsnummer: **0 041 926**
**B1**

(12) **EUROPÄISCHE PATENTSCHRIFT**

(45) Veröffentlichungstag der Patentschrift: **21.11.84**

(51) Int. Cl.³: **C 07 D 209/34**, C 07 D 405/12, C 09 B 26/02

(21) Anmeldenummer: **81810224.6**

(22) Anmeldetag: **05.06.81**

(54) **Verfahren zur Herstellung von Carbinolbasen von Indolinverbindungen.**

(30) Priorität: **11.06.80 CH 4491/80**

(43) Veröffentlichungstag der Anmeldung: **16.12.81 Patentblatt 81/50**

(45) Bekanntmachung des Hinweises auf die Patenterteilung: **21.11.84 Patentblatt 84/47**

(84) Benannte Vertragsstaaten: **CH DE FR GB LI**

(56) Entgegenhaltungen:
**DE - A - 2 325 154**
**GB - A - 1 544 290**

(73) Patentinhaber: **CIBA-GEIGY AG, Postfach, CH-4002 Basel (CH)**

(72) Erfinder: **Zink, Rudolf, Alemannenstrasse 2, CH-4106 Therwil (CH)**
Erfinder: **Loew, Peter, Dr., Concordiastrasse 23, CH-4142 Münchenstein (CH)**

**EP 0 041 926 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von Carbinolbasen von Indolinverbindungen sowie deren Verwendung zur Herstellung von kationischen Verbindungen, und deren konzentrierte wäßrigen Flüssigformulierungen.

Es ist bekannt Carbinolbasen der Formel Ia

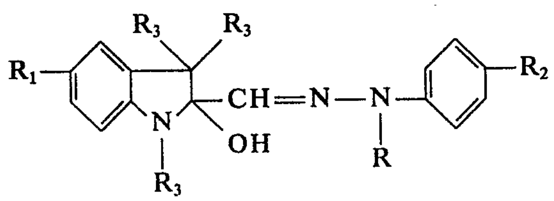

(Ia)

herzustellen worin bedeuten:

R die Methyl- oder Äthylgruppe, $R_1$ Wasserstoff oder Halogen, $R_2$ Wasserstoff, einen $C_1-C_4$-Alkylrest, einen $C_1-C_4$-Alkoxyrest, einen Phenoxyrest, einen Azobenzolrest oder den Rest der Formel $-O-(CH_2)_n-O-R_4$ worin n die Zahlen 1 oder 2 und $R_4$ einen $C_1-C_4$-Alkylrest, den Phenylrest oder auch den $-CH_2$-Rest bedeutet sofern $n=1$ ist und diese $-CH_2$-Gruppe in 0-Stellung zu $R_2$ zu einem 6-Ringsystem verknüpft ist und $R_3$ unabhängig voneinander eine $C_1-C_4$-Alkylgruppe, indem man von kationischen Verbindungen der Formel III

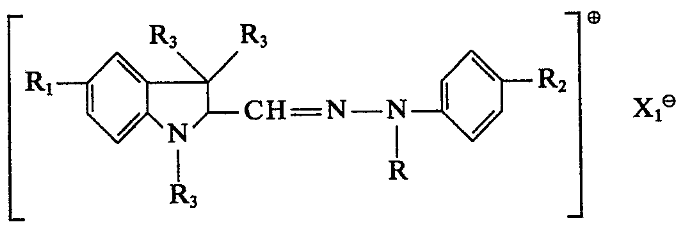

(III)

ausgeht, worin R, $R_1$, $R_2$ und $R_3$ die unter Formel I angegebene Bedeutung haben und $X_1$ ein beliebiges Anion darstellt, und diese mit einer Base z. B. NaOH umsetzt.

$X_1$ in der Bedeutung eines beliebigen Anions stellt z. B. dar: Halogen, wie Chlorid-, Bromid- oder Jodid, Sulfat-, Methylsulfat-, Aminosulfonat-, Carbonat-, Bicarbonat-, Phosphat-, Phosphormolybdat-, Phosphorwolframat-, Phosphorwolframmolybdat-, Benzolsulfonat-, Naphthalinsulfonat-, 4-Chlorbenzolsulfonat- oder komplexe Anionen.

Die kationischen Verbindungen der Formel III, werden dabei z. B. durch eine wäßrige Quaternierung der entsprechenden Farbbasen der Formel IIb

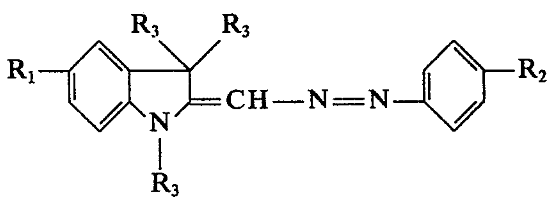

(IIb)

(z. B. aus GB 1 544 290 und DE-OS 2 620 790 bekannt), gewonnen.

Diese Verfahrensweise erfordert somit zwei Schritte um zu den Carbinolbasen zu gelangen, nämlich ein erster Schritt indem man die Farbbase der Formel IIb quaterniert und ein zweiter Schritt, indem man das isolierte Quaternierungsprodukt der Formel III mit einer Base umsetzt.

Die Carbinolbasen besitzen insofern eine Schlüsselstellung, da sie Ausgangsmaterialien für kationische Verbindungen mit einem beliebigen Anion darstellen; vor allem sind Carbonsäuresalze, insbesondere die Acetate nur über dem Weg der Carbinolbasen erhältlich, was bislang derart geschah, daß man aus einer Farbbase der Formel IIb zuerst das Halogenid oder Methosulfat durch Quaternierung herstellt, dieses Quaternierungsprodukt der Formel III isoliert und anschließend zur Carbinolbase umsetzt, gegebenenfalls die Carbinolbase abermals isoliert, aus welcher man dann schließlich das Carbonsäuresalz (z. B. Acetat) der kationischen Verbindung herstellen kann, was total drei Verfahrensschritte erfordert.

Aufgabe der Erfindung war es nun, ein einfacheres und wirtschaftlicheres Verfahren zur Herstellung der Carbinolbasen anzubieten ohne daß dabei kationische Verbindungen als Zwischenprodukte isoliert werden müssen.

Die Lösung dieser Aufgabe besteht in einem einstufigen Verfahren zur Herstellung der Carbinolbasen der Formel I

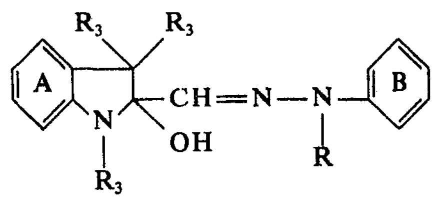

(I)

worin bedeuten:

R die Methyl- oder Äthylgruppe, $R_3$ unabhängig voneinander eine $C_1-C_4$-Alkylgruppe, und worin die Benzolringe A und/oder B gegebenenfalls substituiert sein können, dadurch gekennzeichnet, daß man 1 Mol einer Farbbase der Formel II

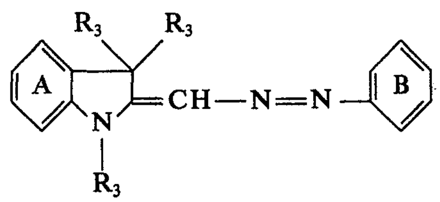

(II)

oder deren Hydrogensalz der Formel IIa

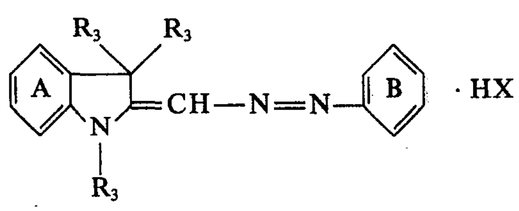

(IIa)

worin $R_3$, A und B die unter Formel I angegebene Bedeutung haben und X ein Anion, vor allem ein Chlorid, Sulfat oder Hydrogensulfat bedeutet, im wäßrig, alkalischen Medium mit einem pH-Wert von mindestens 10 mit mindestens 2 Mol einer den Rest R einführenden Verbindung (Alkylierungsmittel) alkyliert.

In einer bevorzugten Verfahrensweise wird die Alkylierung mit Diäthylsulfat oder vorzugsweise mit Dimethylsulfat durchgeführt, wobei man mindestens 2, insbesondere 2 bis 4, und vorzugsweise mindestens 3 Äquivalente Dimethylsulfat bzw. Diäthylsulfat, bezogen auf die Farbbase der Formel II bzw. IIa verwendet. Die obere Grenze der Anzahl Äquivalente an Alkylierungsmittel ist dabei nicht limitiert; es können auch beispielsweise 100 Äquivalente an Alkylierungsmittel verwendet werden; vielmehr ist die obere Grenze alleine durch die Wirtschaftlichkeit gegeben. Man arbeitet im alkalischen Medium, das man durch Zugabe von Alkalilauge, z. B. NaOH herstellt, wobei der pH-Wert vor oder während der Reaktion gleich oder größer als 10, vorzugsweise 11 ist, bei einer Temperatur von 0 bis 60° C und insbesondere 20—45° C. Nach dieser Arbeitsweise erhält man in einer einstufigen Verfahrensweise in praktisch quantitativer Ausbeute die Carbinolbasen der Formel I. Man geht dabei vorzugsweise von Farbbasen der Formel IIa aus, worin das Symbol X Chlorid, Sulfat oder vor allem Hydrogensulfat bedeutet.

Von besonderem Interesse ist das neue Verfahren zur Herstellung von Carbinolbasen der Formel Ia

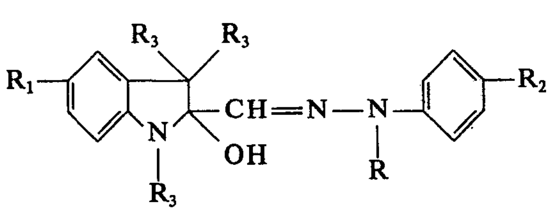

(Ia)

worin bedeuten:

R die Methyl- oder Äthylgruppe, $R_1$ Wasserstoff oder Halogen, $R_2$ Wasserstoff, einen $C_1-C_4$-Alkylrest, einen $C_1-C_4$-Alkylrest, einen $C_1-C_4$-Alkoxyrest, einen Phenoxyrest, einen Azobenzolrest oder den Rest der Formel $-O-(CH_2)_n-O-R_4$ worin n die Zahlen 1 oder 2 und $R_4$ einen $C_1-C_4$-Alkylrest, den Phenylrest oder auch den $-CH_2$-Rest bedeutet sofern n=1 ist und diese $-CH_2$-Gruppe in

0-Stellung zu $R_2$ zu einem 6-Ringsystem verknüpft ist und $R_3$ unabhängig voneinander eine $C_1-C_4$-Alkylgruppe, indem man ein Mol einer Farbbase der Formel IIb

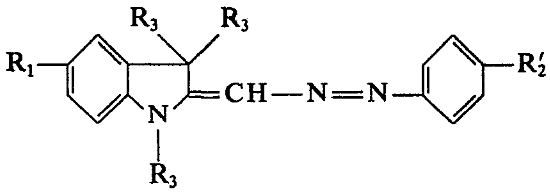

(IIb)

oder deren Hydrogensalz der Formel IIc

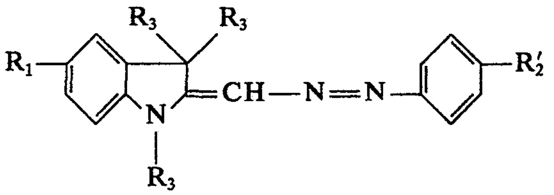

· HX (IIc)

worin $R_1$ und $R_3$ die unter Formel Ia angegebene Bedeutung haben und $R_2'$ die unter Formel Ia angegebene Bedeutung von $R_2$ hat oder OH bedeutet und worin $R_4$ innerhalb von $R_2$ auch H bedeutet und X ein Anion, vorzugsweise Chlorid, Sulfat oder Hydrogensulfat bedeutet, im wäßrig, alkalischen Medium mit einem pH-Wert von mindestens 10 mit mindestens 2 Mol insbesondere 2 bis 4 Mol einer den Rest R einführenden Verbindung vor allem Dimethylsulfat alkyliert.

Bedeutet das Symbol $R_1$ ein Halogenatom, so handelt es sich um das Fluor-, Chlor- oder Bromatom.

Bedeutet $R_2$ eine $C_1-C_4$-Alkylgruppe so handelt es sich um eine unsubstituierte, unverzweigte oder verzweigte Alkylgruppe, beispielsweise um die Methyl-, Äthyl- oder iso-Propylgruppe. Handelt es sich bei $R_2$ um eine $C_1-C_4$-Alkoxygruppe, so kommt vor allem die Methoxy- oder Äthoxygruppe in Frage; bedeutet $R_2$ die $-O-(CH_2)_n-O-R_4$-Gruppierung, so kommen vor allem in Frage: Phenoxymethoxy, Phenoxyäthoxy, (n- und iso-)Butoxymethoxy, (n- und iso) Butoxyäthoxy, (n- und iso) Propoxymethoxy, (n- und iso) Propoxyäthoxy, Äthoxymethoxy, Äthoxyäthoxy und Methoxyäthoxy. Bedeutet $n=1$ so kann $R_4$ auch die Bedeutung von $-CH_2-$ annehmen, wobei diese $-CH_2$-Gruppe zu einem 6-Ring in 0-Stellung zum Substituenten $R_2$ gebunden ist.

Handelt es sich bei $R_3$ um eine Alkylgruppe, so kann diese — unabhängig voneinander — unverzweigt oder verzweigt sein; bevorzugt handelt es sich je um die $CH_3$-Gruppe.

Die erhaltenen Carbinolbasen der Formel I bzw. Ia sind wasserunlöslich und können so vom Reaktionsmedium leicht abgetrennt werden. Sie sind wertvolle Ausgangsmaterialien zur Herstellung von kationischen Verbindungen der Formel III durch Umsetzen mit einer Verbindung $HX_2$ worin $X_2$ ein beliebiges Anion darstellt. Bei $HX_2$ handelt es sich insbesondere um eine Carbonsäure, wie Essigsäure, wobei sich dann die Acetate bilden.

Der große Vorteil des erfindungsgemäßen Verfahrens besteht nun darin, daß auf einfache Art und Weise, z. B. die leicht löslichen Farbstoff-Acetate gewonnen werden können, die für Flüssigformulierungen bestens geeignet sind.

Überraschenderweise sind außerdem mit dem erfindungsgemäßen Verfahren sehr gute Volumenausbeuten und reine Carbinolbasen gewinnbar; die Volumenausbeuten liegen dabei wesentlich günstiger als bei den bekannten Verfahren wo die kationische Verbindung III in die Carbinolbase Ia umgewandelt wird. Schlußendlich ist noch hervorzuheben, daß die Carbinolbase I bzw. Ia direkt aus einer nicht quaternierten Farbbase II bzw. IIa, IIb und IIc hergestellt werden kann.

Die folgenden Beispiele veranschaulichen die Erfindung ohne sie darauf zu limitieren. Teile (T) bedeuten, sofern nichts anderes angegeben ist, Gewichtsteile und die Temperaturen sind in Celsius-Graden angegeben.

Beispiel 1

26,8 T Farbbase der Formel

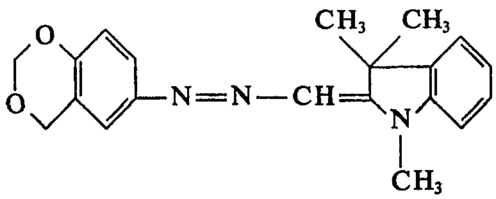

werden bei 20—25° in ein Gemisch von 50 T Wasser und 30,2 T Dimethylsulfat (3 Mol) eingetragen. Bei 25—30° tropft man innert 1 Std. 10,4 T 10 N Natronlauge unter sehr gutem Rühren so zu, daß der pH-Wert des Reaktionsgemisches zwischen 11 und 12 gehalten wird. Hierauf erwärmt man auf 40—45° und hält den pH durch Zugabe weiterer Natronlauge während einer Stunde bei 12. Die gut kristallisierte Carbinolbase der Formel

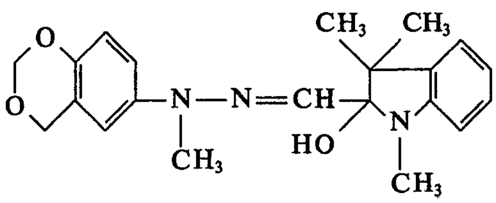

wird abfiltriert, mit 20 T Wasser gewaschen und bei 40° im Vakuum getrocknet. Man erhält 28,5 T eines hellgelben Pulvers.

Beispiel 2

27,5 T des Farbbase-Hydrochlorids der Formel

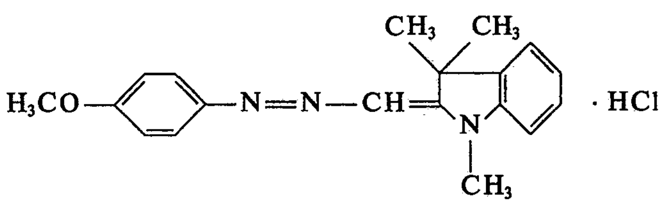

werden bei 20—25° beginnend, in ein Gemisch von 50 T Wasser und 40,3 T Dimethylsulfat (4 Mol) eingetragen, und anschließend wird unter gutem Rühren das Reaktionsgemisch durch Zutropfen von 10 N Natronlauge bei pH 11 gehalten, wobei die Temperatur auf ca. 40° steigt. Die Carbinolbase der Formel

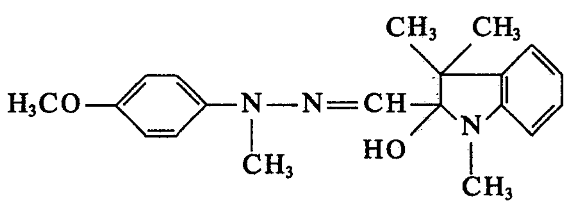

fällt kristallin aus, wird abfiltriert und bei 40° im Vakuum getrocknet. Man erhält 26 T eines gelben Pulvers, das gut löslich in Toluol, und unlöslich in Wasser ist.

Geht man antelle vom HCl-Salz vom $HSO_4$-Salz aus, so erhält man das gleiche Produkt.

Beispiel 3

Das feuchte Nutschgut gemäß Beispiel 2 (=ca. 34 T wasserhaltige Carbinolbase) wird bei 20—25° mit 31 T Eisessig vermischt. Nach kurzer Zeit entsteht eine klare, konzentrierte stabile Farbstofflösung enthaltend das Farbstoffsalz der Formel

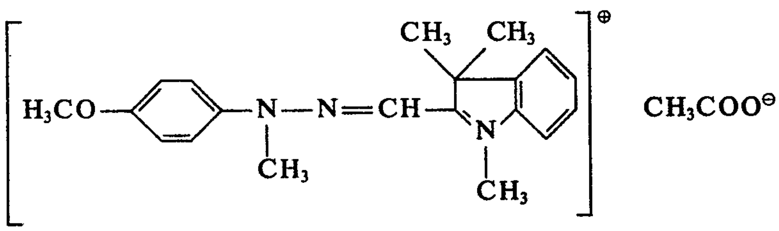

Damit werden auf Polyacrylnitrilfasern goldgelbe Färbungen und Drucke mit hervorragenden Echtheiten erzeugt.

**Patentansprüche**

1. Verfahren zur Herstellung von Carbinolbasen der Formel I

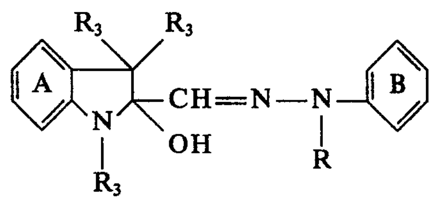

(I)

worin bedeuten:

R die Methyl- oder Äthylgruppe, $R_3$ unabhängig voneinander eine $C_1-C_4$-Alkylgruppe, und worin die Benzolringe A und/oder B gegebenenfalls substituiert sein können, dadurch gekennzeichnet, daß man 1 Mol einer Farbbase der Formel II

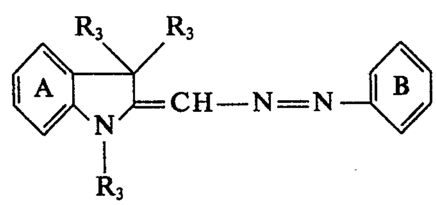

(II)

oder deren Hydrogensalz der Formel IIa

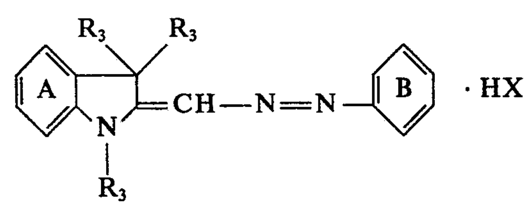

(IIa)

worin $R_3$, A und B die unter Formel I angegebene Bedeutung haben und X ein beliebiges Anion bedeutet, im wäßrig, alkalischen Medium mit einem pH-Wert von mindestens 10 mit mindestens 2 Mol einer den Rest R einführenden Verbindung alkyliert.

2. Verfahren gemäß Anspruch 1, dadurch gekennzeichnet, daß man Carbinolbasen der Formel Ia herstellt,

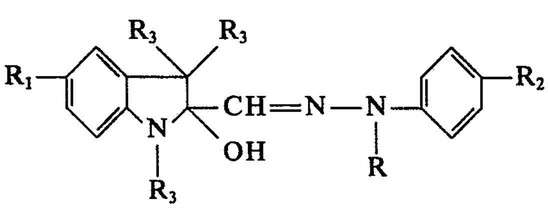

(Ia)

worin bedeuten:

R die Methyl- oder Äthylgruppe, $R_1$ Wasserstoff oder Halogen, $R_2$ Wasserstoff, einen $C_1-C_4$-Alkylrest, einen $C_1-C_4$-Alkoxyrest, einen Phenoxyrest, einen Azobenzolrest oder den Rest der Formel $-O-(CH_2)_n-O-R_4$ worin n die Zahlen 1 oder 2 und $R_4$ einen $C_1-C_4$-Alkylrest, den Phenylrest oder auch den $-CH_2$-Rest bedeutet sofern n = 1 ist und diese $-CH_2$-Gruppe in 0-Stellung zu $R_2$ zu einem 6-Ringsystem verknüpft ist und $R_3$ unabhängig voneinander eine $C_1-C_4$-Alkylgruppe, indem man ein Mol einer Farbbase der Formel IIb

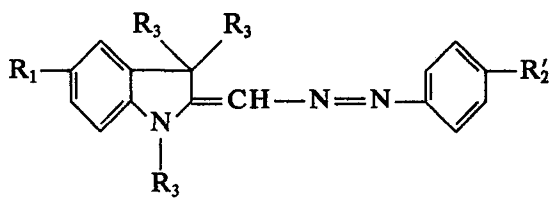

(IIb)

oder deren Hydrogensalz der Formel IIc

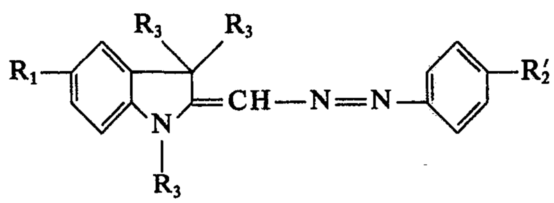

· HX

(IIc)

worin $R_1$ und $R_3$ die unter Formel Ia angegebene Bedeutung haben und $R_2'$ die unter Formel Ia angegebene Bedeutung von $R_2$ hat oder OH bedeutet und worin $R_4$ innerhalb von $R_2$ auch H bedeutet und X ein beliebiges Anion darstellt, im wäßrig, alkalischen Medium mit einem pH-Wert von mindestens 10 mit mindestens 2 Mol einer den Rest R einzuführenden Verbindung alkyliert.

3. Verfahren gemäß den Ansprüchen 1 und 2, dadurch gekennzeichnet, daß das Symbol X ein Chlorid, Sulfat oder Hydrogensulfat bedeutet.

4. Verfahren gemäß den Ansprüchen 1 und 2, dadurch gekennzeichnet, daß als eine den Rest R einführende Verbindung Dimethylsulfat oder Diäthylsulfat verwendet wird.

5. Verfahren gemäß den Ansprüchen 1 bis 4, dadurch gekennzeichnet, daß mindestens 3 Mol Dimethylsulfat oder Diäthylsulfat, bezogen auf ein Mol Farbbase der Formel II bzw. IIb, oder auf das Hydrogensalz der Formel IIa bzw. IIc verwendet werden.

6. Verfahren gemäß den Ansprüchen 1 bis 4, dadurch gekennzeichnet, daß 2 bis 4 Mol Dimethylsulfat, bezogen auf ein Mol Farbbase der Formel II bzw. IIb, oder auf das Hydrogensalz der Formel IIa bzw. IIc verwendet werden.

7. Verfahren gemäß den Ansprüchen 1 bis 6, dadurch gekennzeichnet, daß der pH-Wert des Reaktionsmediums gleich oder größer als 11 ist.

8. Verfahren gemäß Anspruch 1, dadurch gekennzeichnet, daß die Alkylierungsreaktion bei einer Temperatur von 0 bis 60° C durchgeführt wird.

9. Verfahren gemäß Anspruch 8, dadurch gekennzeichnet, daß die Alkylierungsreaktion bei einer Temperatur von 20 bis 45° C durchgeführt wird.

**Claims**

1. A process for the production of a carbinol base of the formula I

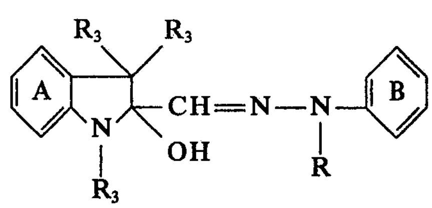

(I)

wherein R is the methyl or ethyl group, each $R_3$ independently is a $C_1-C_4$ alkyl group and the benzene rings A and/or B can be unsubstituted or substituted, which process comprises alkylating 1 mole of a dye base of the formula II

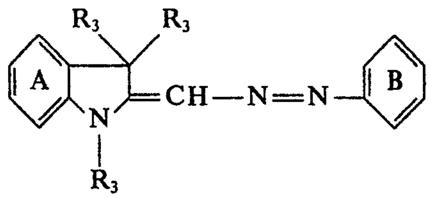

(II)

or the hydrogen salt thereof of the formula IIa

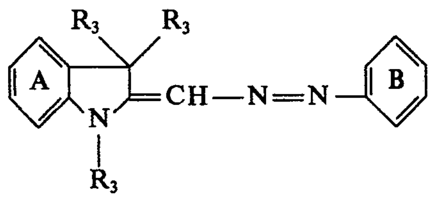

· HX (IIa)

wherein $R_3$, A and B are as defined for formula I and X is any anion, in aqueous alkaline medium having a pH value of at least 10, with at least 2 moles of a compound that introduces the radical R.

2. A process according to claim 1 for the production of a carbinol base of the formula Ia

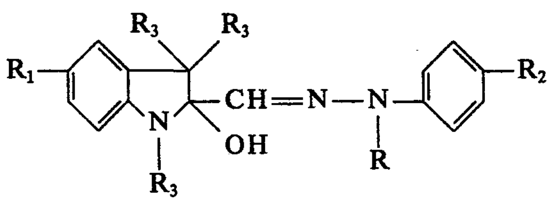

(Ia)

wherein R is the methyl or ethyl group, $R_1$ is hydrogen or halogen, $R_2$ is hydrogen, a $C_1-C_4$ alkyl radical, a $C_1-C_4$ alkoxy radical, a phenoxy radical, an azobenzene radical or a radical of the formula $-O-(CH_2)_n-O-R_4$, wherein n is 1 or 2 and $R_4$ is a $C_1-C_4$ alkyl radical, the phenyl radical or also the $-CH_2-$ radical if n is 1, and said $-CH_2-$ group is attached to a 6-membered ring system in the ortho-position to $R_2$, and each $R_3$ independently is a $C_1-C_4$ alkyl group, which process comprises alkylating 1 mole of a dye base of the formula IIb

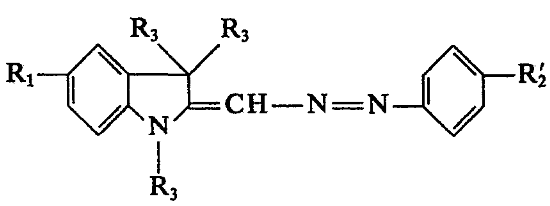

(IIb)

or the hydrogen salt thereof of the formula IIc

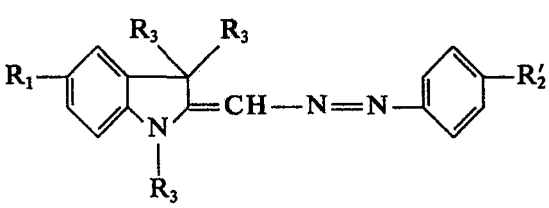

· HX (IIc)

in which formulae $R_1$ and $R_3$ are as defined for formula Ia and $R_2'$ has the meaning of $R_2$ as given for formula Ia or is OH, and in which $R_4$ within the definition of $R_2$ is also hydrogen and X is any anion, in aqueous alkaline medium having a pH of at least 10, with at least 2 moles of a compound that introduces the radical R.

3. A process according to either of claims 1 or 2, wherein X is a chloride, sulfate or hydrogen sulfate anion.

4. A process according to either of claims 1 or 2, wherein the compound employed to introduce the radical R is dimethyl sulfate or diethyl sulfate.

5. A process according to any one of claims 1 to 4, wherein at least 3 moles of dimethyl sulfate or

diethyl sulfate are used, based on 1 mole of the dye base of the formula II or IIb, or on the hydrogen salt of formula IIa or IIc.

6. A process according to any one of claims 1 to 4, wherein 2 to 4 moles of dimethyl sulfate are used, based on 1 mole of the dye base of the formula II or IIb, or on the hydrogen salt of the formula IIa or IIc.

7. A process according to any one of claims 1 to 6, wherein the pH of the reaction medium is 11 or higher than 11.

8. A process according to claim 1, wherein the alkylation is carried out in the temperature range from 0° to 60° C.

9. A process according to claim 8, wherein the alkylation is carried out in the temperature range from 20—45° C.

**Revendications**

1. Procédé pour la préparation de bases carbinoliques de formule I

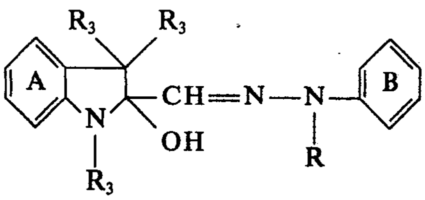

(I)

dans laquelle R est le groupe méthyle ou éthyle; les symboles $R_3$ indépendamment l'un de l'autre sont des groupes $C_1-C_4$ alkyle, et dans laquelle les noyaux benzéniques A et/ou B peuvent être éventuellement substitués, caractérisé par le fait qu'on soumet à l'alkylation une mole d'une base de colorant de formule II

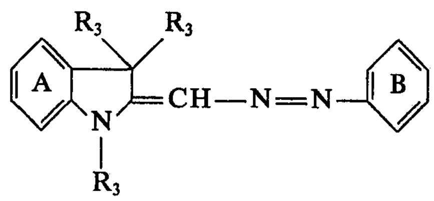

(II)

ou de son sel acide de formule IIa

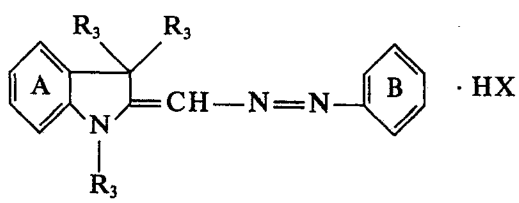

(IIa)

dans lesquelles $R_3$, A et B ont les significations données sous la formule I, et X est un anion quelconque, en milieu alcalin aqueux, à un pH d'au moins 10, avec au moins 2 moles d'un composé introduisant le reste R.

2. Procédé selon la revendication 1, caractérisé par le fait qu'on prépare les bases carbinoliques de formule Ia

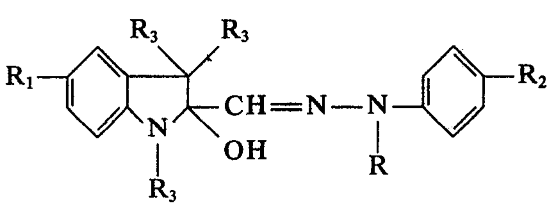

(Ia)

dans laquelle R est le groupe méthyle ou éthyle; $R_1$ est un atome d'hydrogène ou d'halogène; $R_2$ est un atome d'hydrogène, un reste $C_1-C_4$ alkyle, un reste $C_1-C_4$ alcoxy, un reste phénoxy, un reste azobenzénique ou le reste de formule $-O-(CH_2)_n-O-R_4$ où n est le nombre 1 ou 2, et $R_4$ est un reste $C_1-C_4$ alkyle, le reste phényle ou même le reste $-CH_2$ quand n = 1, et ce groupe $-CH_2$ est relié en

position ortho par rapport à $R_2$ à un système hexagonal, et les symboles $R_3$ indépendamment l'un de l'autre sont des groupes $C_1-C_4$ alkyle, en alkylant une mole d'une base de colorant de formule IIb

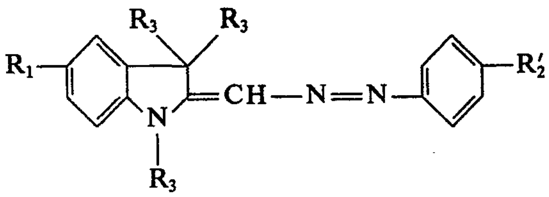

(IIb)

ou de son sel acide de formule IIc

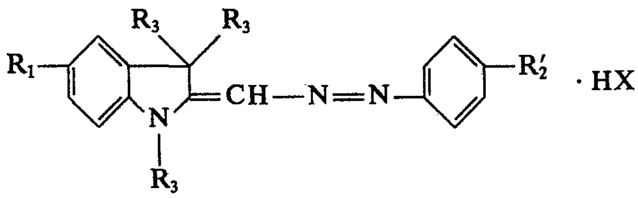

(IIc)

dans laquelle $R_1$ et $R_3$ ont les significations données sous la formule Ia, et $R_2'$ a la signification de $R_2$ donnée sous la formule Ia, ou bien désigne OH, et dans laquelle $R_4$ à l'intérieur de $R_2$ désigne également H, et X est un anion quelconque, en milieu alcalin aqueux, à un pH d'au moins 10, avec au moins 2 moles d'un composé introduisant le reste R.

3. Procédé selon les revendications 1 et 2, caractérisé par le fait que le symbole X désigne un anion chlorure, sulfate ou hydrogénosulfate.

4. Procédé selon les revendications 1 et 2, caractérisé par le fait qu'on utilise comme composé introduisant le reste R le sulfate de diméthyle ou le sulfate de diéthyle.

5. Procédé selon les revendications 1 à 4, caractérisé par le fait qu'on utilise au moins 3 moles de sulfate de diméthyle ou de sulfate de diéthyle par rapport à une mole de la base de colorant de formule II ou IIb, ou bien par rapport au sel acide de formule IIa ou IIc.

6. Procédé selon les revendications 1 à 4, caractérisé par le fait qu'on utilise 2 à 4 moles de sulfate de diméthyle par rapport à une mole de la base de colorant de formule II ou IIb, ou par rapport au sel acide de formule IIa ou IIc.

7. Procédé selon les revendications 1 à 6, caractérisé par le fait que le pH du mélange réactionnel est égal ou supérieur à 11.

8. Procédé selon la revendication 1, caractérisé par le fait que la réaction d'alkylation est effectuée à une température de 0 à 60° C.

9. Procédé selon la revendication 8, caractérisé par le fait que la réaction d'alkylation est effectuée à une température de 20 à 45° C.